(19) Europäisches Patentamt

European Patent Office

Office européen des brevets

(11) **EP 1 384 492 A1**

(12) **EUROPEAN PATENT APPLICATION**

(43) Date of publication:
**28.01.2004 Bulletin 2004/05**

(51) Int Cl.$^7$: **A61N 1/365**, A61N 1/362, A61N 1/368

(21) Application number: **03012264.2**

(22) Date of filing: **11.06.2003**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **22.07.2002 SE 0202288**

(71) Applicant: **St. Jude Medical AB**
**175 84 Järfälla (SE)**

(72) Inventors:
• **Holmström, Nils**
  **175 57 Järfälla (SE)**
• **Björling, Anders**
  **175 53 Järfälla (SE)**
• **Noren, Kjell**
  **171 58 Solna (SE)**
• **Ljungström, Karin**
  **165 70 Hässelby (SE)**
• **Kalling, Sven**
  **183 56 Täby (SE)**

(54) **A heart stimulator**

(57)    A heart stimulator for electric stimulation of a patient's heart comprises an impedance measuring unit (21, 28, 30) adapted to measure the impedance (Z) between at least two measurement electrodes (24, 26) intended to be implanted in a patient such that volume changes of at least one of the chambers of the left heart result in changes in the measured impedance. Analysing means (36) are provided for analysing the measured impedance for the control of the stimulation of the heart. A calculation means (34) is provided to calculate an average impedance morphology curve during a time interval of several cardiac cycles. The analysing means are adapted to analyse the average impedance morphology curve for use for the control of the stimulation to optimise the patient hemodynamics.

Fig. 2

EP 1 384 492 A1

## Description

### *Technical Field*

The present invention relates to a heart stimulator for electric stimulation of a patients heart comprising an impedance measuring unit adapted to measure the impedance between at least two measurement electrodes intended to be implanted in a patient such that volume changes of at least one of the chambers of the left heart result in changes in the measured impedance, and analysing means for analysing said measured impedance for the control of the stimulation of the heart.

### *Background*

Heart stimulators of this general kind are previously known. Thus in US 5 334 422, 5 584 868 and 6 223 079 B1 cardiac stimulating apparatus for use in heart failure therapy are described, wherein intracardiac impedance variations are used for sensing the cardiac function. US 4 535 774 discloses a rate responsive pacer which paces at a rate dependent on detected variations in the stroke volume of the heart. One mentioned example of inferring the stroke volume is by use of impedance measurements. For all these prior art devices the impedance sensing used for controlling the pacing is performed on beat-to-beat bases.

The purpose of the present invention is to propose an improved way of controlling the stimulation timing of a heart stimulator to optimise the patient hemodynamics.

### *Disclosure of the invention*

This purpose is obtained by a heart stimulator of a kind defined in the introductory portion of the description and having the characterizing features of claim 1.

Thus the present invention is using the measured left cardiac impedance averaged over several cardiac cycles for controlling the heart stimulation to optimise hemodynamics.

According to advantageous embodiments of the stimulator according to the invention the impedance measuring unit is adapted to measure real and imaginary parts of impedance and the calculation means is adapted to calculate average values of the real and imaginary parts for use for the control of the stimulation. Blood is resistive and therefore, when the blood volume inside a left heart chamber increases the impedance phase angle will decrease. If, on the contrary, more heart tissue is present the impedance phase angle will become more negative. Because thereof the real and imaginary parts of the measured left cardiac impedance can be used in an advantageous way to optimise hemodynamics of the cardiovascular system. The impedance measuring unit comprises a measuring circuit preferably in the form of a synchronous demodulator for obtaining both the real and imaginary parts of the impedance, and the impedance measuring unit can be adapted to determine the impedance phase angle and the analysing means be adapted to analyse the phase angle for detecting and incipient CHF.

According to other advantageous embodiments of the stimulator according to the invention the analysing means are adapted to analyse at least one predetermined parameter of the average impedance morphology curve for use for the control of the stimulation. A parameter the value which is primarily depending on the left ventricular ejection is preferably used. The parameter is suitably one of the quantities, integrated area below the averaged impedance morphology curve versus time, maximum or minimum value of the average impedance morphology curve, the difference between maximum and minimum values of the average impedance morphology curve, the maximum positive or maximum negative slopes of the average impedance morphology curve, the time between the maximum of the average impedance morphology curve and a predetermined beginning or end of the cardiac cycle.

According to still other advantageous embodiments of the stimulator according to the invention wherein the stimulator is a multisite stimulator, the control means is adapted to control the stimulation timing pattern. Once the optimal stimulation timing pattern is established the stimulation is continuously adjusted to maintain the same pattern. A stimulation pattern can be considered as a vector of different time intervals and the control means can suitably be adapted to first vary the W-interval while keeping AV-and AA-intervals constant till first optimum hemodynamics are obtained, said controlling means being adapted to then keep the W-interval at a constant value equal to the value giving optimum hemodynamics and also keeping the AA-interval constant while varying the AV-interval till second optimum hemodynamics are obtained, said control means being adapted to then keep the AV-interval constant equal to this AV-value giving the second optimum and also keep the W-value at its above mentioned constant value while varying the AA-interval till third optimum hemodynamics are obtained, the control means being adapted to then keep the AA-interval constant equal to this AA-value giving the third optimum and also keep the AV-interval equal to its above mentioned constant AV-value while again varying the W-interval till fourth optimum hemodynamics are obtained, and the control means is adapted to continue this process till the optimum, obtained by the successive variation of these intervals, no longer are improved. It is beneficial to start this process by successively suboptimising the VV-interval, the AV-interval and the AA-interval since changes in the VV-interval will effect the hemodynamics more than changes in the AV-interval, and changes in the AV-interval will effect hemodynamics more than changes in the AA-interval.

In this connection it could also be mentioned that the W timing on patients having Bundle Branch Block is essential for the well being of these patients. It has also been proven that resynchronisation of patients having Left Bundle Branch Block improves there quality of life, e.g. improves the maximum oxygen consumption.

According to yet other advantageous embodiments of the stimulator according to the invention the electrodes are designed for implantation in the right and left atria respectively or for implantation in the right atrium and left ventricle, the impedance thus measured being a measure of the blood volume of left atrium.

According to another advantageous embodiment of the stimulator according to the invention the electrodes intended for the left atrium and the left ventricle are designed for implantation in a coronary vein.

## Brief description of the drawings

To explain the invention in greater detail embodiments chosen as examples of the stimulator according to the invention will be described below with reference to the enclosed drawings, on which figure 1 shows an example of a suitable electrode lead configuration for use in the heart stimulator according to the invention, figure 2 is a block diagram of an embodiment of the heart stimulator according to the invention, figure 3 illustrates the impedance measured across the left ventricle during four cardiac cycles with a first stimulation timing followed by measurements during four cardiac cycles with a second stimulation timing, figure 4 shows averaged left ventricle impedance morphology curves determined for two different stimulation timings and an ECG, figures 5-9 show different stimulation timing patterns as vectors representing different time intervals, and figure 10 illustrate different quantities derivable from measured left cardiac impedance.

## Detailed description of embodiments

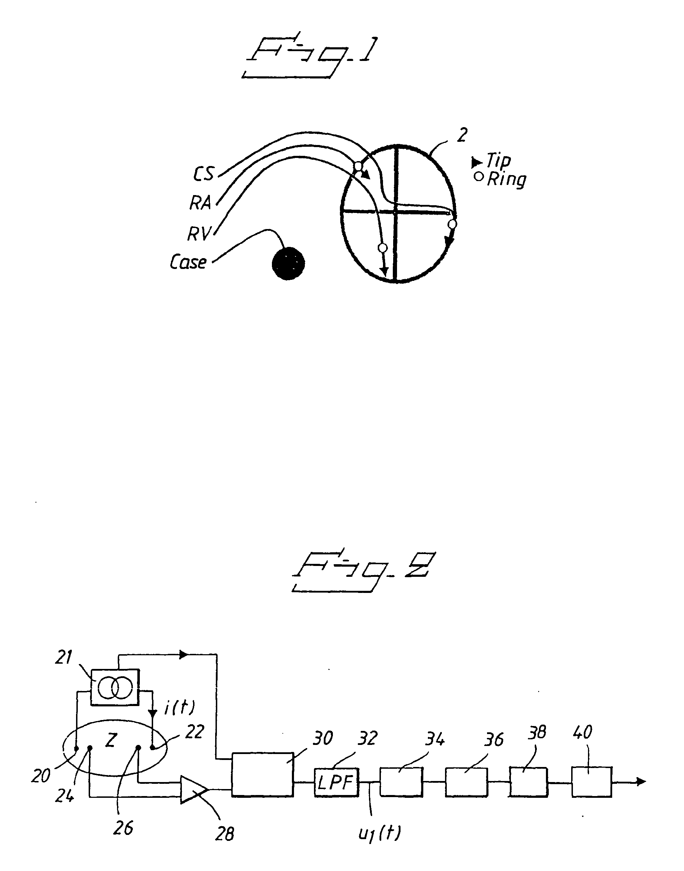

Figure 1 illustrates suitable electrode configurations making impedance measurements possible at several places of the heart 2. By measuring the impedance between left atrium and right atrium RA electrode leads, or between left ventricle and right atrium leads, or possibly between a left atrium lead and the stimulator case, a signal corresponding to the left atrial blood filling is achieved to be used for hemodynamic optimisation of the heart stimulator. A combination of signals obtained from these pairs of electrodes can also be used for this purpose. The left ventricular filling is suitably determined by measuring the impedance between an electrode implanted on the left ventricle and an electrode in the right ventricle. The electrodes intended for left atrium and left ventricle respectively are preferably designed for implantation in a coronary vein in CS. To get a good fixation of the electrode it is beneficial to use a screw-in electrode.

As discussed in the co-pending Swedish application number ... excessive filling of blood in the left atrium and left ventricle can originate from several different cardiac dysfunctions. According to the invention this can be detected by impedance measurements, and the information obtained from this impedance measurement is used for controlling the stimulation such that hemodynamics is improved by curing left heart filling pattern.

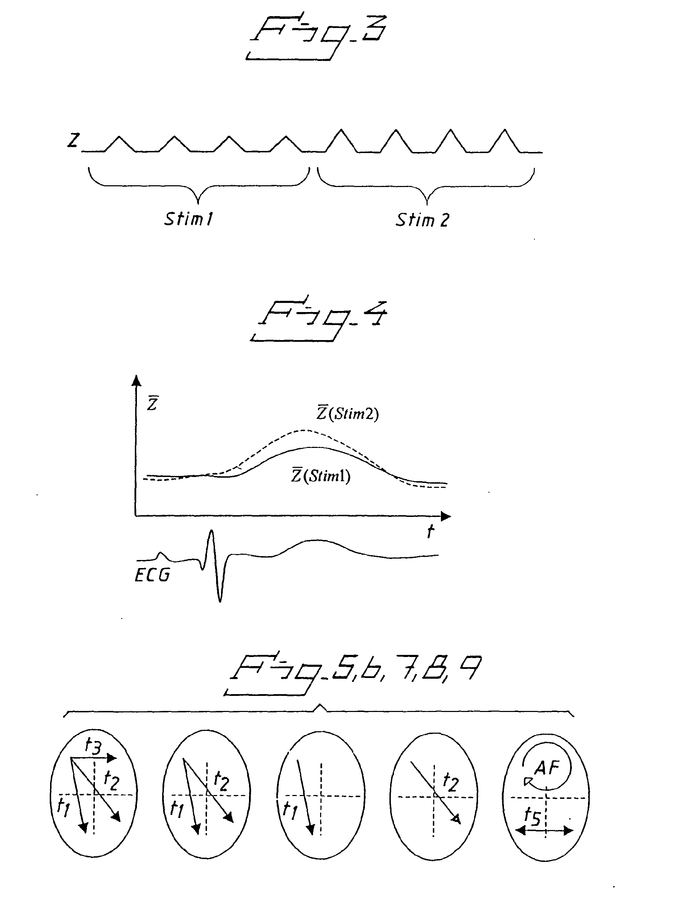

Figure 2 is a block diagram of an embodiment of the heart stimulator according to the invention. A current i(t) is delivered from a current source 21 to electrodes 20, 22 and the evoked voltage response is measured between electrodes 24, 26. The evoked voltage response is amplified in amplifier 28 and with the aid of a reference signal picked up from the current source 21 synchronised in a multiplier 30 with the current. A low pass filter 32 is used to obtain an average voltage signal $U_1(t)$. The corresponding average impedance curve $Z_1$ is given by

$$Z_1 = u_1/i$$

The average impedance morphology curve $Z_1$ obtained from calculation means 34 is analysed in analysing means 36. A control means 38 is connected to the analysing means 36 to control the heart stimulator pulse generator 40 in response to the output from the analysing means 36 such that patient hemodynamics are optimized.

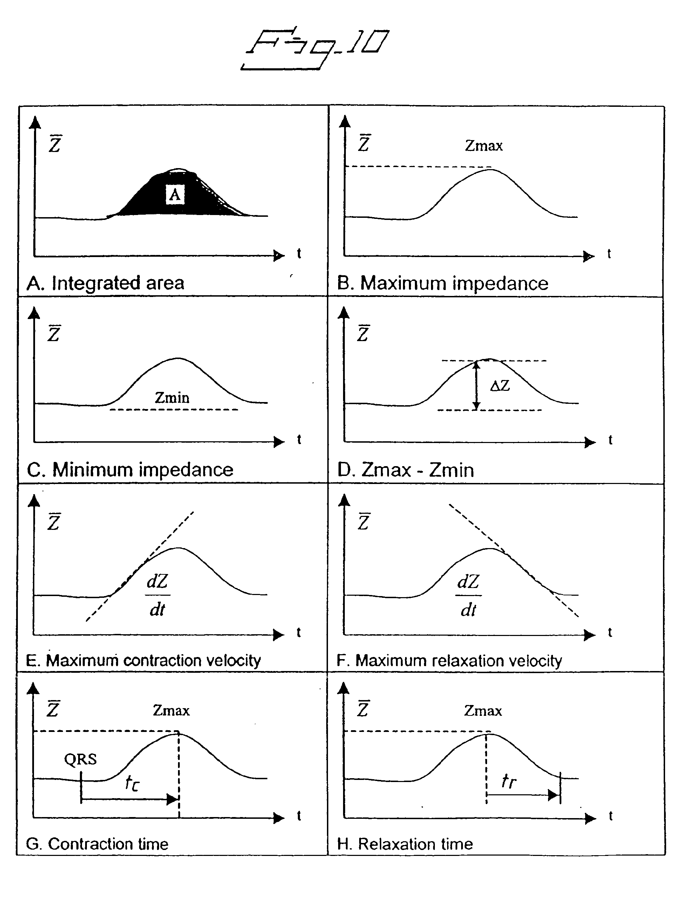

Figure 3 illustrates the impedance continuously measured across the left ventricle. The sampling frequency is high enough to make it possible to follow variations during each cardiac cycle, e.g. equal to 128 Hz. During the first four cardiac cycles shown in the figure stimulation is performed according to a first stimulation timing pattern Stim1, and during the four subsequent cardiac cycles the stimulation timing pattern Stim2 is changed.

Figure 4 shows the average impedance morphology curve $\bar{Z}$ (Stim1) for a cardiac cycle, calculated during a time interval with a fixed stimulation timing pattern Stim1. This time interval includes several cardiac cycles, e.g. 10-100 cycles. Thereafter one or more of the timing parameters are changed and a new average impedance morphology curve $\bar{Z}$ (Stim2) is calculated for a similar time interval. The ECG curve is also shown in the figure.

A quantity depending on the left ventricular ejection is calculated from the average impedance morphology curves, $\bar{Z}$ cf. figure 10 below. By comparing one of these quantities, e.g. the integrated area A, see figure 10, for the two timings patterns Stim1 and Stim2 the most favourable of this two timing patterns is selected, and this process can be continued for different stimulation timing patterns Stim such that the area A is maximised as an indication of a maximum stroke volume.

The stimulation timing pattern can be described as a vector of different time intervals $(t_1, t_2, t_3)$, $(t_1, t_2)$ etc., where $t_1$ is the time from right atrial T-wave detection to stimulation in the right ventricle, see figures 5-7. The parameter $t_1$ can also be the time from a right atrial stimulation to right ventricular stimulation which should be selected independently from sensed events. Parameters $t_2$ and $t_3$ denote corresponding right atrium to left ventricle and right atrium to left atrium times respectively. Thus the parameters $t_1$, $t_2$ and $t_3$ define the AA-, AV- and W-intervals, the parameters $t_1$ and $t_2$ and the quantity $t_2 - t_3$ defining three different AV-intervals, as appears from figure 5.

If it is not possible to stimulate or sense in the left atrium a three chamber embodiment according to figure 6 is selected. If the patient suffers from atrial defribillation or atrial tachycardia only the two ventricles are involved in the optimisation procedure, cf. figure 9.

In figure 7 the right AV-delay is optimised with reference to left ventricle blood filling. A coronary sinus lead is necessary to measure the left ventricle impedance.

Figure 8 illustrates a situation where a left ventricular lead is used only for measurement of the impedance Z.

Figure 10 shows eight examples of quantities, which can be derived from the measured left ventricular impedance. These quantities are integrated area A below the average impedance morphology curve $\bar{Z}$ versus time t, maximum and minimum values $Z_{max}$ and $Z_{min}$ of the average impedance morphology curve $\bar{Z}$ diagrams B and C in the figure, the difference $\Delta Z$ between maximum and minimum values of the average impedance morphology curve $\bar{Z}$, diagram D in the figure, the maximum positive or maximum negative slopes dZ/dt of the average impedance morphology curve $\bar{Z}$, diagrams E and F in the figure, and the time between the maximum $Z_{max}$ of the average impedance morphology curve $\bar{Z}$ and a predetermined beginning (QRS) or end of thecardiac cycle, diagrams G and H in the figure. The maximum positive slope according to diagram E in figure 10 represents maximum contraction velocity of the left ventricle and the maximum negative slope according to diagram F in figure 10 represents the maximum relaxation velocity. The time $t_c$ according to diagram G and the time $t_r$ according to diagram H in figure 10 represent the contraction and the relaxation times respectively of the left ventricle.

With the technique according to present invention a relatively stable optimum stimulation timing pattern is obtained which only needs to be checked or verified at, preferably regular, intervals.

Besides the measured cardiac impedance's other quantities and conditions can also be considered for use in optimising the stimulation timing pattern. Thus e.g. rate and activity conditions as well as respiratory minute volume can be of importance in connection with optimisation of the stimulation timing pattern.

## Claims

1. A heart stimulator for electric stimulation of a patient's heart comprising an impedance measuring unit (21, 28, 30) adapted to measure the impedance (Z) between at least two measurement electrodes intended to be implanted in a patient such that volume changes of at least one of the chambers of the left heart result in changes in the measured impedance, and analysing means (36) for analysing said measured impedance for the control of the stimulation of the heart, **characterized in that** a calculation means (34) is provided to calculate, for a predetermined stimulation pattern, an average impedance morphology curve during a time interval of several cardiac cycles, and **in that** said analysing means (36) are adapted to analyse said average impedance morphology curve for use for the control of the stimulation to optimise the patient hemodynamics.

2. The stimulator according to claim 1, **characterized in that** said impedance measuring unit (21, 24, 26, 28) is adapted to measure real and imaginary parts of the impedance (Z) and **in that** said calculation means (34) is adapted to calculate average values of said real and imaginary parts for use for the control of the stimulation.

3. The stimulator according to claim 2, **characterized in that** said impedance measuring unit comprises a measuring circuit in the form of a synchronous demodulator (20, 21, 22, 24, 26, 28, 30) for obtaining both the real and imaginary parts of the impedance (Z).

4. The stimulator according to claims 2 or 3, **characterized in that** said impedance measuring unit is adapted to determine the impedance phase angle and **in that** said analysing means (36) are adapted to analyse said phase angle for detecting an incipient CHF.

5. The stimulator according to claim 1, **characterized in that** said analysing means (36) are adapted to analyse at least one predetermined parameter (A, $Z_{max}$, $Z_{min}$, $\Delta Z$, dZ/dt, $t_c$, $t_r$) of said average impedance morphology curve (Z) for use for the control of the stimulation.

6. The stimulator according to any of the preceding claims, **characterized in that** said impedance measuring unit (21, 28, 30) is adapted to sample the measured impedance with a sufficiently high frequency to be able to follow impedance variations during a cardiac cycle.

7. The stimulator according to claims 5 or 6, **characterized in that** said parameter is one of the quantities, integrated area (A) below the average impedance morphology curve versus time, maximum ($Z_{max}$) or minimum ($Z_{min}$) value of the average impedance morphology curve, the difference ($\Delta Z$) between maximum and minimum values of the average impedance morphology curve, the maximum positive or maximum negative slopes (dZ/dt) of the average impedance morphology curve, the time between the maximum of the average impedance morphology curve and a predetermined beginning ($t_c$) or end ($t_r$) of the cardiac cycle.

8. The stimulator according to claim 7, **characterized in that** a control means (38) is connected to said analysing means (36) for controlling the stimulation to optimize at least one of said parameters from the patient hemodynamics' point of view.

9. The stimulator according to claim 8, said parameter being said integrated area, **characterized in that** said control means (38) is adapted to control the stimulation such that said area (A) is at a maximum.

10. The stimulator according to claims 7 or 8, **characterized in that** said control means (38) s adapted to analyse a combination of said parameters for use for the control of the stimulation.

11. The stimulator according to any one of the claims 8-10, said stimulator being a multisite stimulator, **characterized in that** said control means (38) is adapted to control the stimulation timing pattern (Stim).

12. The stimulator according to claim 9, **characterized in that** said control means (38) is adapted to first vary the W-interval while keeping AV-and AA-intervals constant till first optimum hemodynamics are obtained, said controlling means being adapted to keep the W-interval at this constant value and also keeping the AA-interval constant while varying the AV-interval till second optimum hemodynamics are obtained, said control means being adapted to then keep the AV-interval constant equal to this AV-value and also keep the W-value at its above mentioned constant value while varying the AA-interval till third optimum hemodynamics are obtained, said control means being adapted to then keep the AA-interval constant equal to this AA-value and also keep the AV-interval at its above mentioned constant AV-value while varying the VV-interval till fourth optimum hemodynamics are obtained, and **in that** said control means is adapted to continue this process till said optimum hemodynamics no longer are improved.

13. The stimulator according to any one of the claims 8-12, **characterized in that** said control means (38) is adapted to control the stimulation rate.

14. The stimulator according to any one of the preceding claims, a pulse generator (40) being provided to deliver stimulation pulses to the patient's heart through implantable stimulation electrodes, **characterized in that** said measurement electrodes (20, 22, 24, 26) are forming said stimulation electrodes.

15. The stimulator according to any one of the claims 1-13, a pulse generator (40) being provided to deliver stimulation pulses to the patient's heart through implanted stimulation electrodes, **characterized in that** at least one of said stimulation electrodes is different from said measurement electrodes (20, 22, 24, 26).

16. The stimulator according to any one of the preceding claims, characterized in that said electrodes are designed for implantation in the right and left atria, respectively.

17. The stimulator according to any one of the claims 1-15, **characterized in that** said electrodes are designed for implantation in the right atrium and left ventricle.

18. The stimulator according to any one of the claims 1 -15, **characterized in that** one of said electrodes is designed for implantation in the left atrium and the other electrode is formed of an outer case of the stimulator.

19. The stimulator according to any one of the preceding claims, characterized in that said electrodes intended for the left atrium and the left ventricle are designed for implantation in a coronary vein (CS).

Fig.1

Fig.2

Fig.3

Fig.4

Fig.5, 6, 7, 8, 9

Fig. 10

A. Integrated area

B. Maximum impedance

C. Minimum impedance

D. Zmax - Zmin

E. Maximum contraction velocity

F. Maximum relaxation velocity

G. Contraction time

H. Relaxation time

**European Patent**
**Office**

## PARTIAL EUROPEAN SEARCH REPORT

which under Rule 45 of the European Patent Convention
shall be considered, for the purposes of subsequent
proceedings, as the European search report

Application Number

EP 03 01 2264

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | US 6 223 079 B1 (DE ROOS COBUS ET AL) 24 April 2001 (2001-04-24) * abstract; figures 4-6 * * column 3, line 38 - column 4, line 33 * * column 8, line 1 - column 9, line 61 * --- | 1 | A61N1/365 A61N1/362 A61N1/368 |
| Y | EP 0 615 770 A (CARDIAC PACEMAKERS) 21 September 1994 (1994-09-21) * abstract; figure 4 * * page 3, line 42 - page 4, line 10 * * page 5, line 14 - line 49 * ----- | 1 | |

**TECHNICAL FIELDS SEARCHED (Int.Cl.7)**

A61N

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 13 November 2003 | Wetzig, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

**European Patent Office**

**INCOMPLETE SEARCH
SHEET C**

Application Number

EP 03 01 2264

Claim(s) searched completely:
        1

Claim(s) not searched:
        2-19

Reason for the limitation of the search:

Claim 1 does not meet the requirements of Articles 52(1) EPC and 56 EPC, since its subject-matter cannot be considered to involve an inventive step in view of a combination of documents US-B-6 223 079 and EP-A-0 615 770.

In view of the large number of dependent claims directly depending on independent claim 1 (non-patentable due to lack of an inventive step), which render it difficult, if not impossible, to determine the matter for which protection is sought, the present application fails to comply with the clarity and conciseness requirements of Article 84 EPC to such an extent that a meaningful search of claims 2-19 is impossible.

**ANNEX TO THE EUROPEAN SEARCH REPORT
ON EUROPEAN PATENT APPLICATION NO.**

EP 03 01 2264

This annex lists the patent family members relating to the patent documents cited in the above-mentioned European search report.
The members are as contained in the European Patent Office EDP file on
The European Patent Office is in no way liable for these particulars which are merely given for the purpose of information.

13-11-2003

| Patent document cited in search report | | Publication date | Patent family member(s) | | Publication date |
|---|---|---|---|---|---|
| US 6223079 | B1 | 24-04-2001 | US | 6070100 A | 30-05-2000 |
| | | | AU | 1714499 A | 05-07-1999 |
| | | | EP | 1039951 A1 | 04-10-2000 |
| | | | WO | 9930777 A1 | 24-06-1999 |
| | | | US | 6223082 B1 | 24-04-2001 |
| | | | US | 6219579 B1 | 17-04-2001 |
| | | | US | 6238420 B1 | 29-05-2001 |
| | | | US | 2001010009 A1 | 26-07-2001 |
| EP 0615770 | A | 21-09-1994 | CA | 2091708 A1 | 17-09-1994 |
| | | | US | 5235976 A | 17-08-1993 |
| | | | EP | 0615770 A1 | 21-09-1994 |
| | | | DE | 69323543 D1 | 25-03-1999 |
| | | | DE | 69323543 T2 | 08-07-1999 |
| | | | ES | 2130222 T3 | 01-07-1999 |
| | | | AU | 3522493 A | 29-09-1994 |